# EUROPEAN PATENT SPECIFICATION

(11) **EP 3 665 294 B1**
(45) Date of publication and mention of the grant of the patent: **23.10.2024**
(21) Application number: 18765177.3
(22) Date of filing: 06.08.2018
(51) Int. Cl.: C12P 7/44

(54) **GENETICALLY MODIFIED CANDIDA MALTOSA FOR THE PRODUCTION OF DICARBOXYLIC FATTY ACIDS**
GENETISCH MODIFIZIERTE CANDIDA MALTOSA ZUR HERSTELLUNG VON DICARBONFETTSÄUREN
CANDIDA MALTOSA GÉNÉTIQUEMENT MODIFIÉ POUR LA PRODUCTION D'ACIDES GRAS DICARBOXYLIQUES

(30) Priority: 09.08.2017 IT 201700091114
(43) Date of publication of application: 17.06.2020
(73) Proprietor: Novamont S.p.A., 28100 Novara (IT)
(72) Inventor: PERINI, Davide, 28068 Romentino (NO) (IT); PAGLINO, Alessandra, 28010 Caltignaga (NO) (IT); SCARDUA, Alessandro, 20853 Biassono (MB) (IT)
(74) Representative: Studio Torta S.p.A.
(86) International application number: PCT/IB2018/055907
(87) International publication number: WO 2019/030652

(56) References cited:
- WO-A2-99/04014
- SABINE HUF ET AL: "Biotechnological synthesis of long-chain dicarboxylic acids as building blocks for polymers", EUROPEAN JOURNAL OF LIPID SCIENCE AND TECHNOLOGY., vol. 113, no. 5, 13 April 2011 (2011-04-13), DE, pages 548 - 561, XP055309008, ISSN: 1438-7697, DOI: 10.1002/ejlt.201000112
- CAO ET AL: "Engineering the acetyl-CoA transportation system of candida tropicalis enhances the production of dicarboxylic acid", BIOTECHNOLOGY JOURNAL, WILEY-VCH VERLAG, WEINHEIM, DE, vol. 1, 1 January 2006 (2006-01-01), pages 68 - 74, XP002397012, ISSN: 1860-6768, DOI: 10.1002/BIOT.200500008
- REUSZ O ET AL: "The SAT1 flipper, an optimized tool for gene disruption in Candida albicans", G, ELSEVIER, AMSTERDAM, NL, vol. 341, 27 October 2004 (2004-10-27), pages 119 - 127, XP004596109, ISSN: 0378-1119, DOI: 10.1016/J.GENE.2004.06.021
- LIHUA ZHANG ET AL: "Development of an efficient genetic manipulation strategy for sequential gene disruption and expression of different heterologous GFP genes in Candida tropicalis", APPLIED MICROBIOLOGY BIOTECHNOLOGY, vol. 100, 2016, pages 9567 - 9580, XP002780555

## Description

### TECHNICAL FIELD

This invention relates to a genetically modified microorganism of the species *Candida maltosa* for the production of long chain diacids from monocarboxylic acids characterised in that the genetic modification comprising inactivation of both alleles of the CAT (Carnitine Acetyl-Transferase) gene, that is a gene which does not take part in the β-oxidation metabolic route, is carried out on *wild type* microorganism.

### BACKGROUND ART

Medium-long chain dicarboxylic acids (in the meaning of this invention, from 10 carbon atoms upwards) have a vast range of applications: from use as monomers for the synthesis of polymers to use in various industries such as cosmetics, pharmaceuticals, and plant protection products. For example, sebacic acid (C10) is used for the synthesis of polyesters, which have applications in fibres, films, resins, plasticisers, synthetic lubricants and adhesives. Producing them by a chemical route is however technically difficult, not environmentally very sustainable and economically expensive.

In the last 20 years research has therefore turned its attention to the production of dicarboxylic acids through biotechnological processes carried out by microorganisms capable of using alkanes, oils or fatty acids as substrates, in order to obtain sustainable low-cost processes.

In particular, fatty acids are metabolised via the β-oxidation pathway so that inhibition or deletion of the genes which take part in this metabolic pathway will result in reduced degradation of fatty acids, whereas in microorganisms provided with metabolic pathways for the functionalisation of fatty acids (e.g. ω-oxidation) it will result in increased conversion of such compounds. For example, US 5,254,466 describes complete blocking of the β-oxidation pathway in *Candida tropicalis* achieved through deletion of the POX genes, which makes it possible to obtain dicarboxylic acids by a fermentation route.

Alternative strategies to block the β-oxidation pathway, through which the β-oxidation metabolic pathway is not blocked directly but its functionality is inhibited, are also described in the literature via deletion of the CAT gene in *Candida tropicalis.* This restricts the transport of acetyl-CoA (a derivative from the degradation of fatty acids via β-oxidation) between peroxisomes and mitochondria, consequently inhibiting β-oxidation functionality. For example, Cao et al., Biotechnol. J., 1:68-74 (2006) describes a homozygote strain of *Candida tropicalis* with inactivation of the two alleles of the CAT gene by gene disruption. However, the homozygote *Δcat* strain shows no production of dicarboxylic acids and produces less cellular biomass than the original strain in the presence of glucose as the sole carbon source.

Furthermore, Lihua Zhang et al., Appl. Microbiol. Biotechnol., 100:9567-9580 (2016) describes strains of *Candida tropicalis* with deletion of the two alleles of the CAT gene which were previously subjected to random mutagenesis leading to a favourable genetic background and amplifying the effect of the deletions of both the alleles. In this case, the mutated strain from which the mutant originated with deletion of the two alleles is in fact already capable of producing quantities of dodecanedioic (C₁₂) acid from dodecane.

WO 99/04014 A2 discloses a process for producing dicarboxylic acids using genetically engineered Candida maltosa. The Candida maltosa has gene disruptions in the 8-oxidation pathway by knocking out the POX4 genes encoding for isoenzymes of the acyl CoA oxidase. SABINE HUF ET AL: "Biotechnological synthesis of long-chain dicarboxylic acids as building blocks for polymers", EUROPEAN JOURNAL OF LIPID SCIENCE AND TECHNOLOGY, vol. 113, no. 5, 13 April 2011, pages 548-561, and CAO ET AL: "Engineering the acetyl-CoA transportation system of candida tropicalis enhances the production of dicarboxylic acid", BIOTECHNOLOGY JOURNAL, WILEY-VCH VERLAG, WEINHEIM, DE, vol. 1, 1 January 2006, pages 68-74 disclose genetically engineered strains of C. tropicalis, in which the CAT gene encoding for carnitine-acetyl transferase has been deleted.

REUSZ 0 ET AL: "The SAT1 flipper, an optimized tool for gene disruption in Candida albicans", G, ELSEVIER, AMSTERDAM, NL, vol. 341, 27 October 2004, pages 119-127, refers to a method of gene deletion in C. albicans using a deletion cassette.

LIHUA ZHANG ET AL: "Development of an efficient genetic manipulation strategy for sequential gene disruption and expression of different heterologous GFP genes in Candida tropicalis", APPLIED MICROBIOLOGY BIOTECHNOLOGY, vol. 100, 2016, pages 9567-9580 relates to C. tropicalis strains subjected to random mutagenesis and subsequent deletion of one or both alleles of the CAT gene.

### DISCLOSURE OF INVENTION

This invention instead relates to a process for the production of dicarboxylic acids from monocarboxylic acids through a *wild type* microorganism *Candida maltosa* provided with the ω-oxidation metabolic pathway and having the peroxisome and mitochondrial carnitine acetyl-transferase coded by a single gene, characterized by inactivation of the function of the CAT (Carnitine Acetyl-Transferase) gene, in which that inactivation is done by making non-functional both the alleles of the CAT (Carnitine Acetyl-Transferase) gene, and/or the product of its transcription, without the microorganism being subjected to random mutagenesis techniques that are expensive and of insecure outcome.

For the purpose of the invention, the term *"Candida maltosa"* is used according to its usual meaning as it distinguishes from *Candida tropicalis* in that they are two distinct species in the field of yeast taxonomy and show significant differences at the molecular and biochemical level. See, for instance, Meyer et al., Arch. Microbiol., 104:225-231 (1975), Kanekok et al., Agric. Biol. Chem., 41 (11):2269-2275 (1977), and C.P Kurtzman et al., The yeasts, a Taxonomic study, Volume 2, Fifth edition, 1135-1136 and 1256-1258 (2011).

In particular, this invention relates to the development of a strain of *wild type Candida maltosa* through deletion of both the alleles of the CAT (Carnitine Acetyl-Transferase) gene, the said deletion making it possible to restrict intracellular transport of acetyl-coenzyme A deriving from the dissipative metabolism of fatty acids, thus therefore inhibiting the dissipative pathway itself and encouraging the ω-oxidation metabolic pathway, with consequent conversion to dicarboxylic fatty acids. The CAT gene has been inactivated by double crossing over recombination with a deletion cassette.

This invention is also particularly advantageous because of the fact that *Candida maltosa* is included in biological risk class 1, while other known microorganisms used for the production of dicarboxylic acids and belonging to the *Candida* genus are included in biological risk class 2 (e.g. *Candida tropicalis*)*.*

The *wild type* microorganism *Candida maltosa* used in the process of the invention is advantageously a (*CAT*/*CAT*) strain, in particular a CGMCC 2.1386 strain.

Carnitine acetyltransferase is an enzyme belonging to the class of transferases and catalyses the following reaction: acetyl-CoA + carnitine ⇄ CoA + acetylcarnitine.

Acetyl-coenzyme A (acetyl-CoA), which is subsequently used in the glyoxylate cycle and in the tricarboxylic acid cycle, is produced during metabolism of the fatty acids. Within eukaryotic cells some of these processes take place in well-defined compartments or organelles, such as in peroxisomes (e.g. the glyoxylate cycle), mitochondria (e.g. the tricarboxylic acids cycle) or in the cytosol, and it is therefore necessary to ensure intracellular transport of acetyl-CoA.

Movement of acetyl-CoA is facilitated by enzymes of the carnitine acetyltransferase (CAT) family which catalyse transport of the acetyl group of the acetyl-CoA to carnitine with the consequent production of acetylcarnitine (and *vice versa*) that is subsequently transported across the membrane. It is thought that the peroxisome CAT enzyme is involved in exporting acetyl-CoA produced during β-oxidation and that the main function of mitochondrial CAT is associated with the release of acetyl-CoA from acetyl carnitine in the mitochondrion. Peroxisome carnitine acetyl-transferase and the mitochondrial type are generally coded by a single gene. Although some microorganisms are strictly dependent on CAT for the movement of acetyl-CoA, others (such as *S*. *cerevisiae*) showed two parallel metabolic pathways dependent on CAT or peroxisome citrate synthase (Cit2).

In the meaning of this invention, by inactivation of the CAT gene is meant any technique which makes non-functional both the alleles of the CAT gene or the product of its transcription or the product of its translation. The genetic technique of inactivation by deletion is particularly preferred. Even more preferred is the genetic technique of deletion performed by a process of double crossing over recombination, in particular using a deletion cassette. For the purposes of this invention, a plasmid containing a gene for resistance to an antibiotic under the control of a constitutive promoter and a gene for recombinase under the control of an inducible promoter is particularly preferred for allowing the deletion of both the alleles of the CAT gene.

### BEST MODE FOR CARRYING OUT THE INVENTION

### EXAMPLES

A *wild type* strain (*CAT*/*CAT*) of *Candida maltosa* CGMCC2.1386 (BNCC340010=BNCC140359; hereinafter indicated as strain '010) from the BNBIO microorganisms collection as characterised in the table was used to create the mutant with the deletion of 2 alleles of the CAT gene (*cat*/*cat*)*. Candida maltosa* '010 grows in the presence of oleic acid as the sole carbon source but does not produce dicarboxylic acids.

| ***Glucose*** | ***Oleic acid*** | ***Glucose and subsequent addition of oleic acid*** |
|---|---|---|
| Growth | Growth | Growth with glucose but without producing DCA |

A Sigma-Aldrich technical grade oleic acid having the following composition - oleic acid 91.1%, linoleic acid 4.5%, stearic acid 2.6%, palmitic acid 1.8% - was used as substrate. The strain subsequently underwent genetic modifications through a process of double crossing over recombination using a suitably constructed deletion cassette to allow the deletion of the CAT gene, at first partly (1 deleted allele, the so-called *cat*/*CAT* mutant) and then completely (2 deleted alleles, the so-called *cat*/*cat* mutant). The deletion cassette was constructed starting from a pSFS2 plasmid developed for deleting genes in the species *Candida albicans.* The plasmid contains:
- the gene for resistance to an antibiotic under the control of a constitutive promoter,
- the gene for a recombination under the control of an inducible promoter.

The two genes mentioned are located between two FRT (Flippase Recognition Target) sequences. Recombinase is an enzyme promoting recombination between FRT sites, substantially eliminating the sequences lying between them. Expression of recombinase therefore makes it possible to eliminate the gene for resistance to the antibiotic once the gene has been deleted, making it possible to reuse the same antibiotic/plasmid for a further deletion. The nucleotide sequence of the gene coding for peroxisome and mitochondrial CAT in *C*. *tropicalis* was used to search for the homologous sequence in the genome of *C*. *maltosa* Xu316 deposited in the NCBI database by BLAST software. The nucleotide sequence of the CAT gene in *C*. *maltosa* Xu316 was in turn used to design the primers to amplify the CAT gene in *C*. *maltosa* '010 and corresponding portions of interest by PCR (Polymerase Chain Reaction). As far as deletion of the first allele of the CAT gene is concerned, a deletion cassette was constructed using the plasmid described above and the regions in 5' (Flanking Region 1) and 3' (Flanking Region 2) in the CAT gene amplified by PCR and subsequently inserted into the plasmid. The deletion cassette constructed in this way was used to transform the *C*. *maltosa* '010 strain and the antibiotic resistant clones with one deleted allele were identified by PCR. In order to be able to delete the second allele of the CAT gene, the recombinase was expressed to eliminate resistance to the antibiotic; this step was performed for one clone showing that one CAT allele was deleted. The clones which then were sensitive to the antibiotic were analysed by PCR and one clone from which the gene for resistance to the antibiotic had been excised was then transformed by a new deletion cassette.

This new deletion cassette was constructed using the plasmid described above and a different region in 5' (Flanking Region 3) of the CAT gene, while the region in 3' corresponds to Flanking Region 2; these regions were amplified by PCR and subsequently inserted into the plasmid. The clones transformed with the construct in which the second allele was deleted were then selected as reported above and identified by PCR.

Finally, the fragment containing resistance to the antibiotic was removed as indicated above and the definitive clone was evaluated using PCR allowing the identification of a strain of C. *maltosa* '010 in which both the alleles of the CAT gene were deleted. Results for the first preliminary comparative tests between the wt and *cat*/*CAT* and *cat*/*cat* strain are shown below.

### Flask growth test

| | ***Sole source of carbon*** | |
|---|---|---|
| ***Strain*** | ***Glucose*** | ***Oleic acid*** |
| '010 *CAT*/*CAT*(*wt*) | ++ | ++ |
| '010 *cat*/*CAT* | ++ | +/- |
| '010 *cat*/*cat* | ++ | - |

### BRIEF DESCRIPTION OF THE DRAWINGS

### Plate growth test

Glucose as the sole source of carbon.

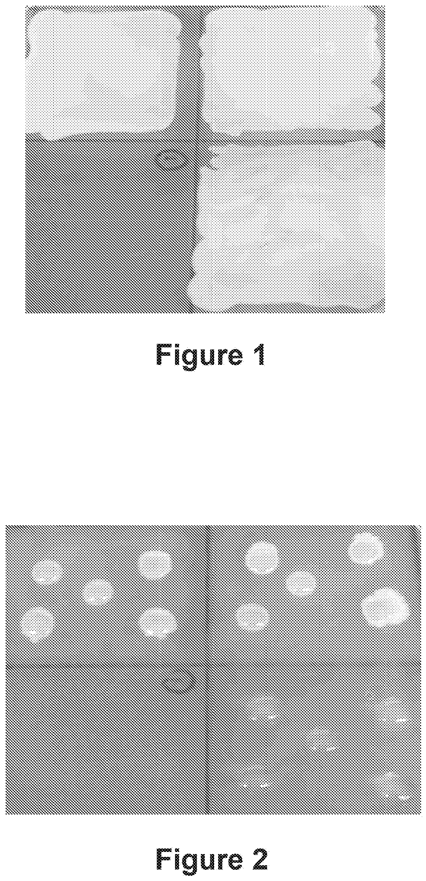

See Figure 1.

### Oleic acid as the sole source of carbon.

See Figure 2.

It is clear that the strain of *Candida maltosa* '010 in which both the alleles of the CAT gene were deleted ('010 *cat*/*cat*) is unable to grow using oleic acid as the sole source of carbon, thus suggesting that the modification of the metabolic pathway involving the use of acetyl-CoA has an inhibiting effect on the β-oxidation pathway.

Subsequently, the fermentation performance of the *Candida maltosa* '010 *cat*/*cat* strain in comparison with the '010 *CAT*/*CAT* (*wt*) strain was evaluated under different process conditions. The process provides for a two-stage fermentation with uncoupling between the stage of cell growth to biomass (with consumption of glucose initially provided in the substrate) and the stage of producing dicarboxylic acids from mixtures of monocarboxylic acids.

The technical oleic acid used as substrate had the following composition: oleic acid 91.1%, linoleic acid 4.5%, stearic acid 2.6%, palmitic acid 1.8%.

| **Strain** | **Substrate** | **Feeding rate of oleic acid** | **Feeding of syrup [g/L/h]** | **Production time (from added substrate)** | **1,18-octadecenedioi c [g/L]** |
|---|---|---|---|---|---|
| '010 *cat*/*cat* | Rich A6 | low | 1.5 to approximately 35 hours, then interruption | 78 hours | **1.1** |
| '010 *CAT*/*CAT* | Rich A6 | high | 1.5 for approximately 35 hours, then interruption | 78 hours | **0** |

| | | | | | |
|---|---|---|---|---|---|
| Rich A6: Composition: phosphate buffer; yeast extract (5 g/L), source of nitrogen (3.81 g/L), vitamin (1.0 mg/L), magnesium salts (0.6 g/L), 1.25 ml of trace elements (Teknova TE 800x), glucose (> 50 g/L). | | | | | |

### Feeding of oleic acid

Low = feeding rate controlled to maintain the substrate concentration at approximately 20 g/L for the duration of the production stage.

High = feeding rate controlled to maintain the substrate concentration at approximately 50 g/L for the duration of the production stage. The high feeding rate ensures a supply of fatty acids which is consistent with a wt microorganism capable of mainly consuming fatty acids via the β-oxidation metabolic pathway thus allowing the transport of derived acetyl-CoA through the "carnitine shuttle" process.

Fermentation samples were collected and analysed, first by Gas Chromatography using a Flame Ionisation Detector (GC-FID) and subsequently by Gas Chromatography associated with Mass Spectrometry (GC-MS). On the basis of the GC-FID data the output of dicarboxylic acid was 1.1 g/L in the case of the *Candida maltosa* '010 *cat*/*cat* samples. GC-MS analysis subsequently confirmed that the peak quantified in GC-FID actually corresponded to 1,18-octadecenedioic acid.

The *Candida maltosa* '010 *cat*/*cat* strain therefore shows a clear tendency to produce dicarboxylic acids (approximately 1.1 g/L), providing evidence of the presence of an oxidative metabolic pathway (ω-oxidation of fatty acids) which is favoured under conditions in which both the alleles of the CAT gene are deleted. Conversely, under the same conditions, the *Candida maltosa* '010 wt strain does not produce dicarboxylic acids.

## Claims

1. Process for the production of dicarboxylic acids from monocarboxylic acids, through a wild type microorganism *Candida maltosa* having the ω-oxidation metabolic pathway and having the peroxisome and mitochondrial carnitine acetyl-transferase coded by a single gene, **characterized by** inactivation of the function of the CAT (Carnitine Acetyl-Transferase) gene, in which that inactivation is done by making non-functional both the alleles of the CAT gene, and/or the product of its transcription.

2. Process according to claim 1, in which the inactivation stage is a deletion stage.

3. Process according to claim 2, in which the deletion stage takes place through a process of double crossing over recombination.

4. Process according to claim 3, in which deletion makes use of a deletion cassette.

5. Process according to claim 4, in which a first deletion cassette brings about the deletion of one allele of the CAT gene and a second deletion cassette brings about deletion of the other allele of the CAT gene.

6. Process according to any one of claims 1-5, in which the wild type microorganism is a *Candida maltosa* CGMCC 2.1386 strain.

7. Genetically modified strain of *Candida maltosa* in which both the alleles of the CAT gene have been inactivated by double crossing over recombination with a deletion cassette.

8. Genetically modified strain of *Candida maltosa* according to claim 7, in which recombination is brought about using a plasmid containing a gene for resistance to an antibiotic under the control of a constitutive promoter and a gene for a recombinase under the control of an inducible promoter.

9. Genetically modified strain of *Candida maltosa* according to claim 7 or 8, in which both the alleles of the CAT gene of a *Candida maltosa* CGMCC 2.1386 wild type strain have been inactivated by double crossing over recombination with a deletion cassette.

10. Use of *Candida maltosa* according to claim 8 or 9 for the production of dicarboxylic acids from a source of monocarboxylic acids.

## Patentansprüche

1. Verfahren zur Herstellung von Dicarbonsäuren aus Monocarbonsäuren durch einen Wildtyp-Mikroorganismus *Candida maltosa aufweisend* den m-Oxidations-Stoffwechselweg und aufweisend die peroxisomale und mitochondriale Carnitin-Acetyltransferase, die durch einem einzigen Gen kodiert wird, **gekennzeichnet durch** Inaktivierung der Funktion des CAT-Gens (Carnitin-Acetyltransferase), wobei diese Inaktivierung erfolgt, indem beide Allele des CAT-Gens, und/oder das Produkt seiner Transkription funktionsunfähig gemacht werden.

2. Verfahren nach Anspruch 1, wobei die Inaktivierungsstufe eine Deletionsstufe ist.

3. Verfahren nach Anspruch 2, wobei die Deletionsstufe durch einen Prozess der doppelten Überkreuzungsrekombination erfolgt.

4. Verfahren nach Anspruch 3, wobei die Deletion eine Deletionskassette verwendet.

5. Verfahren nach Anspruch 4, wobei eine erste Deletionskassette die Deletion eines Allels des CAT-Gens bewirkt und eine zweite Deletionskassette die Deletion des anderen Allels des CAT-Gens bewirkt.

6. Verfahren nach einem der Ansprüche 1 bis 5, wobei der Wildtyp-Mikroorganismus ein *Candida maltose Stamm* CGMCC 2.1386 ist.

7. Gentechnisch veränderter Stamm von *Candida maltosa,* wobei beide Allele des CAT-Gens mittels doppelter Überkreuzungsrekombination mit einer Deletionskassette inaktiviert worden sind.

8. Genetisch veränderter Stamm von *Candida maltosa* nach Anspruch 7, wobei die Rekombination unter Verwendung eines Plasmids erfolgt, das ein Gen für die Resistenz gegen ein Antibiotikum unter der Kontrolle eines konstitutiven Promotors und ein Gen für eine Rekombinase unter der Kontrolle eines induzierbaren Promotors enthält.

9. Gentechnisch veränderter Stamm von *Candida maltosa* nach Anspruch 7 oder 8, wobei beide Allele des CAT-Gens eines *Candida maltosa* Wildtyp Stamm CGMCC 2.1386 mittels doppelter Überkreuzungsrekombination mit einer Deletionskassette inaktiviert wurden.

10. Verwendung von *Candida maltosa* nach Anspruch 8 oder 9 zur Herstellung von Dicarbonsäuren aus einer Quelle von Monocarbonsäuren.

## Revendications

1. Procédé de production d'acides dicarboxyliques à partir d'acides monocarboxyliques, par le biais d'un micro-organisme de type sauvage *Candida maltosa* ayant la voie métabolique de la m-oxydation et dont le peroxysome et la carnitine acétyl-transférase mitochondriale sont codés par un seul gène, **caractérisé par** l'inactivation de la fonction du gène CAT (Carnitine Acetyl-Transferase), dans lequel cette inactivation est faite en rendant non fonctionnels les deux allèles du gène CAT et/ou le produit de sa transcription.

2. Procédé selon la revendication 1, dans lequel l'étape d'inactivation est une étape de suppression.

3. Procédé selon la revendication 2, dans lequel l'étape de suppression a lieu par un processus de recombinaison à double croisement.

4. Procédé selon la revendication 3, dans lequel la suppression se fait à l'aide d'une cassette de suppression.

5. Procédé selon la revendication 4, dans lequel une première cassette de suppression entraîne la suppression d'un allèle du gène CAT et une seconde cassette de suppression entraîne la suppression de l'autre allèle du gène CAT.

6. Procédé selon l'une des revendications 1 à 5, dans lequel le micro-organisme de type sauvage est une souche *Candida maltose* CGMCC 2.1386.

7. Souche génétiquement modifiée de *Candida maltosa* dans laquelle les deux allèles du gène CAT ont été inactivés par double recombinaison avec une cassette de suppression.

8. Souche génétiquement modifiée de *Candida maltosa* selon la revendication 7, dans laquelle la recombinaison est réalisée à l'aide d'un plasmide contenant un gène de résistance à un antibiotique sous le contrôle d'un promoteur constitutif et un gène de recombinase sous le contrôle d'un promoteur inductible.

9. Souche génétiquement modifiée de *Candida maltosa* selon la revendication 7 ou 8, dans laquelle les deux allèles du gène CAT d'une *Candida maltosa* CGMCC 2.1386 souche de type sauvage ont été inactivés par double recombinaison avec une cassette de suppression.

10. Utilisation de *Candida maltosa* selon la revendication 8 ou 9 pour la production d'acides dicarboxyliques à partir d'une source d'acides monocarboxyliques.
